# EUROPEAN PATENT SPECIFICATION

(11) **EP 2 505 123 B1**
(45) Date of publication and mention of the grant of the patent: **10.03.2021**
(21) Application number: 12161183.4
(22) Date of filing: 26.03.2012
(51) Int. Cl.: A61B 1/12, A61B 1/32, A61B 1/015, A61M 13/00

(54) **Endoscope air-supply system**
Endoskopisches Luftversorgungssystem
Système d'alimentation en air d'endoscope

(30) Priority: 28.03.2011 JP 2011070293
(43) Date of publication of application: 03.10.2012
(73) Proprietor: FUJIFILM Corporation, Tokyo 106-8620 (JP); Osaka University, Suita-shi, Osaka 565-0871 (JP)
(72) Inventor: Torisawa, Nobuyuki, Kanagawa, 258-8538 (JP); Nakajima, Kiyokazu, Osaka, 565-0871 (JP)
(74) Representative: Klunker IP Patentanwälte PartG mbB

(56) References cited:
- EP-A2- 0 689 793
- GB-A- 2 198 948
- US-A- 4 464 169
- US-A- 5 439 441
- US-A1- 2005 222 535

## Description

### BACKGROUND OF THE INVENTION

### Field of the Invention

The present invention relates to an endoscope air-supply system according to the preamble of claim 1, and in particular to an endoscope air-supply system to supply constant-pressure gas from an air-supply device into a lumen of a subject through an opening provided at a distal end portion of a flexible endoscope inserted into the lumen to perform observation or treatment of the lumen.

### Description of the Related Art

Conventionally, in the field of medicine, medical diagnosis utilizing an endoscope is widely performed, in particular, an image of a lumen of a subject is taken by a built-in imaging device, such a CCD, at an inserted distal end of an endoscope inserted in the lumen, and signals for the image are subjected to processing by a processor and the processed image is displayed on a monitor so that a doctor can observe the image to use the image for diagnosis or to insert a treatment instrument through a channel for treatment instrument passage to perform a treatment, such as sample collection or polypectomy.

At this time, in order to secure the field of view of an observation window provided at the distal end portion of the endoscope, or secure a space for manipulating the treatment instrument, gas is supplied to the lumen from the air-supply device through an air-supply duct provided for the endoscope, but it is necessary to keep measuring the pressure of gas to maintain the gas at a proper pressure, for example, so as not to make the gas pressure so low that the lumen deflates to cause difficulty in observation or treatment, or otherwise so as not to make the gas pressure so high as to put a strain on a patient.

Therefore, the Japanese Patent Application Laid-Open No. 2006-288881, for example, discloses that when a flexible endoscope is inserted into the lumen of the large intestine or the like and carbon dioxide gas is supplied to the lumen by an air-supply system in order to observe the lumen, timing of a timer is started when the supply of carbon dioxide gas to the lumen is started, and the supply is stopped when a timed value reaches a set value so that carbon dioxide gas is saved.

Further, the Japanese Patent Application Laid-Open No. 2003-250886 discloses a pneumoperitoneum device with an air-supply connector capable of being connected with a pneumoperitoneum needle or a trocar for pneumoperitoneum through an air-supply tube so that the pneumoperitoneum needle or the trocar for pneumoperitoneum punctures the abdomen of a patient, thereby injecting gas into the abdominal cavity, wherein a pressure sensor that detects the pressure of the gas is provided in an internal duct of the pneumoperitoneum device, and in the case of using the trocar with low duct resistance, the pressure sensor measures a stable pressure after a certain period of time, and in the case of using the pneumoperitoneum needle with high duct resistance, based upon such recognition that the pneumoperitoneum needle is used, when the flow rate of the gas, including a fluctuation in flow rate, is equal to or less than a certain value, measurement of the flow rate or pressure of the gas is further delayed.

### SUMMARY OF THE INVENTION

As described above, conventionally, for example, as disclosed in the patent literature 1, in order to secure the field of view of the endoscope when a lumen of a subject, such as the stomach, the large intestine, or the esophagus, is observed by the endoscope, the lumen is inflated by supplying gas through the air-supply duct of the endoscope. Further, in order to ease patient's pain at this time, carbon dioxide gas, which is quickly absorbed by a living body, is used as gas to inflate the lumen.

Conventionally, however, an operator operates an air-supply button manually to supply gas to the lumen, and there is the problem that the operator needs to operate the air-supply button frequently in order to inflate the lumen and keep the pressure in the lumen constant.

Further, it is necessary to measure the gas pressure in the lumen in order to keep the gas pressure in the lumen appropriate, but there is the problem that attachment of body fluid to the air-supply duct communicating with the pressure sensor, in particular to an opening portion thereof, makes it impossible to measure the gas pressure in the lumen correctly.

Further, as shown in the Japanese Patent Application Laid-Open No. 2003-250886, in the field of surgery, endoscopic observation or operation is performed while the abdominal cavity is inflated with a certain pressure by the pneumoperitoneum device to secure a surgical field, but unlike the lumen described above such a phenomenon as luminal obstruction does not usually occur in the abdominal cavity, and therefore no special control on the phenomenon is required.

In accordance with the preamble of claim 1 GB 2 198 948 A discloses an endoscope air-supply system in which in addition to a constant-supply duct a suction duct connected to a suction device is provided, wherein the pressure measurement device continuously measures pressure within the lumen. The suction device is activated if the measured pressure is higher than a predetermined pressure in order to obtain a substantially constant pressure within the lumen. A manually operated valve is connected between the suction channel and the suction device so as to suck debris and the like from the space at the distal end of an endoscope through the suction channel. Similarly, when the suction channel is clogged by debris, the measured pressure increases thereby activating the suction device which is sucks debris from the suction channel.

A similar endoscope air-supply system is known from EP 0 689 793 A2. An additional feature shown in this document provides for freezing the display indicating the measured pressure in case that an abnormally high pressure is measured, caused by debris included in the suction channel. Freezing of the display is made in order to take into account that an abnormal high pressure is not due to abnormal high pressure within the lumen

Another air-supply system is known from US 5,439,441, where insufflation gas is introduced into a body cavity to be inflated, then the gas flow is stopped, and the pressure in the body cavity is measured at plural, discrete intervals of time.

The present invention has been made in view of these circumstances, and an object of the present invention is to provide an endoscope air-supply system which does not require frequent operation for inflating a lumen when an endoscope is inserted into the lumen to perform observation or an operation, and which can measure pressure in the lumen correctly and perform air supply so as to keep the pressure in the lumen appropriate.

In order to achieve the object, a first aspect of the present invention defined in claim 1 provides an endoscope air-supply system including a gas supply device which supplies predetermined gas to a lumen of a subject through an air-supply duct; a pressure measurement device which measures pressure in the lumen and which is connected through a duct for pressure measurement communicating with the lumen; a flushing device which supplies gas for flushing to the duct for pressure measurement; and an instruction device which instructs the pressure measurement device to perform pressure measurement and which instructs the flushing device to supply the gas for flushing in synchronism with the pressure measurement by the pressure measurement device.

Thereby, since flushing is performed in synchronism with pressure measurement, thereby removing attached matter, such as liquid, in the duct for pressure measurement so that the pressure in the lumen can be measured precisely, air supply can be performed so as to keep the pressure appropriate, and an operator does not need to perform frequent operation of inflating the lumen when an endoscope is inserted into the lumen to perform observation or treatment. In the first aspect of the present invention, the instruction device instructs the pressure measurement device to perform pressure measurement during stoppage of gas supply by the gas supply device, and instructs the flushing device to supply the gas for flushing before the pressure measurement device performs pressure measurement.

Since flushing is performed before pressure measurement in this manner, attached matter in the duct for pressure measurement is removed so that precise pressure can be measured.

Further, according to a second aspect of the present invention, the flushing device supplies an amount of the gas for flushing equivalent to or more than the volume of the duct for pressure measurement.

This makes it possible to remove attached matter in the duct for pressure measurement with a small amount of gas supply, and therefore the need to supply a large amount of gas to the lumen is eliminated so that the strain on the subject can be eased.

Further, according to a third aspect of the present invention, the duct for pressure measurement is provided with a check valve which prevents backflow of fluid from the lumen, and the flushing device supplies the gas for flushing which is at least equal to a volume from the check valve to an opening portion on the side of the lumen of the duct for pressure measurement.

This makes it possible to perform duct flushing air supply with a volume smaller than the total volume of a duct for constant-pressure air supply.

Further, according to a fourth aspect of the present invention, the duct for pressure measurement is composed of at least a portion of the air-supply duct.

Further, according to a fifth aspect of the present invention, the gas for flushing supplied by the flushing device is gas supplied from the same gas supply source as the gas supplied by the gas supply device.

Since gas supplied from the same gas supply source as the gas supplied by the gas supply device is used for the gas for flushing supplied by the flushing device so as to utilize a portion of the air-supply duct in this manner, a configuration of the apparatus can be simplified.

Further, according to a sixth aspect of the present invention, the endoscope air-supply system further includes a first control device which controls gas supply performed by the gas supply device based on the result of the measurement by the pressure measurement device.

Since the gas is supplied based on the result of pressure measurement in this manner, more appropriate gas supply can be performed.

Further, according to an seventh aspect of the present invention, the endoscope air-supply system further includes a second control device which controls the flow velocity or the flow rate of the gas for flushing supplied by the flushing device based on a condition of supply of the gas supplied by the gas supply device.

Since the gas for flushing is controlled based on a condition of gas supply in this manner, more appropriate control of flushing can be performed.

Further, according to a eighth aspect of the present invention, the second control device controls the flow velocity of the gas for flushing supplied by the flushing device so as to be equal to the flow velocity of the gas supplied by the gas supply device.

Thus, the flow velocity of the gas for flushing may be equal to the flow velocity of the gas supplied by the gas supply device.

Further, according to a ninth aspect of the present invention, the second control device controls the flow velocity of the gas for flushing supplied by the flushing device so as to be faster than the flow velocity of the gas supplied by the gas supply device.

When flushing is performed at a faster flow velocity than that of the gas supplied by the gas supply device in this manner, flushing can be performed at a lower flow rate for a shorter time.

Further, according to an tenth aspect of the present invention, the instruction device issues an instruction to repeat supply of the gas for flushing by the flushing device and pressure measurement by the pressure measurement device in plural times during stoppage of gas supply by the gas supply device.

Since pressure measurement and flushing are performed in plural times in this manner, pressure measurement can be performed more precisely.

Further, according to a eleventh aspect of the present invention, the endoscope air-supply system further includes a determination device which determinates whether or not a difference between the result of Nth pressure measurement and the result of (N + 1)th pressure measurement is less than a predetermined threshold (where N is a natural number equal to or more than 1) during stoppage of gas supply by the gas supply device, wherein the instruction device issues an instruction to repeat supply of the gas for flushing by the flushing device and pressure measurement by the pressure measurement device until the determination device determines that the difference is less than the predetermine threshold.

Since flushing and pressure measurement are repeated until the difference between plural measurement results becomes less than a predetermined value in this manner, more precise pressure measurement can be performed.

Further, according to a twelfth aspect of the present invention, the endoscope air-supply system further includes a display device by which whether or not supply of the gas for flushing by the flushing device or gas supply by the gas supply device is being performed can be confirmed.

This makes it possible for an operator to always know what air supply the endoscope system is currently performing, so that operation efficiency is improved. Advantageous Effect of the Invention.

As described above, according to the present invention, intraluminal pressure can be precisely measured, thereby performing air supply so as to keep an appropriate pressure, and when an endoscope is inserted into a lumen to perform observation or treatment, an operator does not need to perform frequent operation of inflating the lumen.

### BRIEF DESCRIPTION OF THE DRAWINGS

Fig. 1 is a configuration diagram showing the outline of an endoscopic system provided with an endoscope air-supply system according to a first embodiment of the present invention;
Fig. 2 is a perspective view showing a distal end portion of an insertion part of an endoscope;
Fig. 3 is a configuration diagram showing the outline of an air-supply device of the first embodiment;
Fig. 4 is a diagram showing a first example of a constant-pressure air-supply control method;
Fig. 5 is a diagram showing a second example of the constant-pressure air-supply control method;
Fig. 6 is a diagram showing a third example of the constant-pressure air-supply control method;
Fig. 7 is a configuration diagram showing the outline of an endoscopic system provided with an endoscope air-supply system according to a second embodiment of the present invention;
Fig. 8 is a configuration diagram showing the outline of an air-supply device of the second embodiment; and
Fig. 9 is a configuration diagram showing the outline of an air-supply device of a modification of the second embodiment.

### DETAILED DESCRIPTION OF THE PREFERRED EMBODIMENTS

Hereinafter, with reference to the accompanying drawings, an endoscope air-supply system according to the present invention will be described in detail.

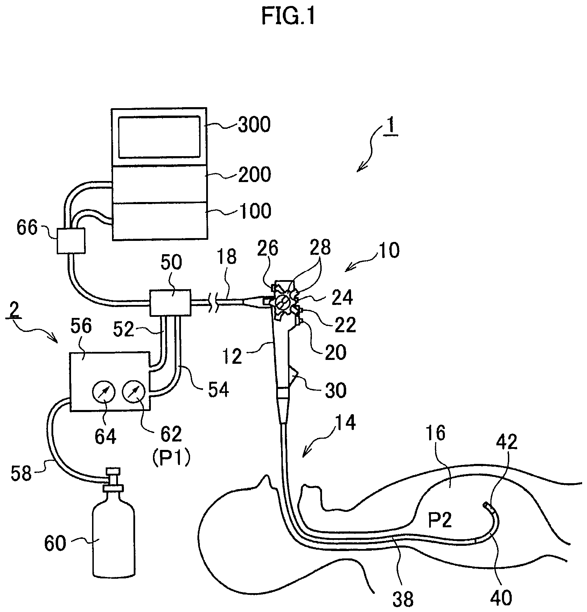

Fig. 1 is a configuration diagram showing the outline of an endoscopic system provided with an endoscope air-supply system according to a first embodiment of the present invention.

As shown in Fig. 1, an endoscopic system 1 is provided with an endoscope air-supply system 2. The endoscopic system 1 is mainly composed of an endoscope (flexible endoscope) 10, an endoscope air-supply system 2, a light source device 100, an endoscope processor 200, and a monitor device (display device) 300.

The endoscope 10 is provided with a hand operation part 12 and an insertion part 14 provided so as to be continuous with the hand operation part 12. An operator grasps the hand operation part 12 disposed on a proximal end side of the endoscope 10 to operate the endoscope 10, and inserts a distal end side of the insertion part 14 into a lumen of a subject, such as a stomach 16, to perform observation, diagnosis, or curative treatment.

The hand operation part 12 is connected with a universal cable 18, and the universal cable 18 is provided with an air-supply connector 50. The air-supply connector is connected with two air-supply tubes (a tube for air supply and water supply 52 and a tube for constant-pressure air supply 54), and these air-supply tubes (the tube for air supply and water supply 52 and the tube for constant-pressure air supply 54) are connected with an air-supply device (gas supply device) 56.

Further, a distal end of the universal cable 18 is provided with an LG connector 66. This LG connector 66 is attachably and detachably coupled with the light source device 100, thereby transmitting illumination light to an illumination optical system (not shown) disposed at a distal end portion of the insertion part 14. Further, the LG connector 66 is connected with an electrical connector through the universal cable 18, and the electrical connector is attachably and detachably coupled with the endoscope processor 200. This outputs data of an observation image obtained by the endoscope 10 to the endoscope processor 200, and the observation image is displayed on the monitor device 300 connected with the endoscope processor 200.

Further, the hand operation part 12 is provided with an air-supply and water-supply button 20, a suction button 22, a shutter button 24, a seesaw switch for zooming 26, angle knobs 28, and a forceps entrance 30.

The air-supply device 56 is coupled with a carbon dioxide gas cylinder 60 through a tube for high-pressure gas 58. Liquefied carbon dioxide gas is stored in the carbon dioxide gas cylinder 60. The carbon dioxide gas stored in the carbon dioxide gas cylinder 60 is reduced (adjusted) to a predetermined pressure by the air-supply device 56, and then supplied through the tube for air supply and water supply 52 and the tube for constant-pressure air supply 54.

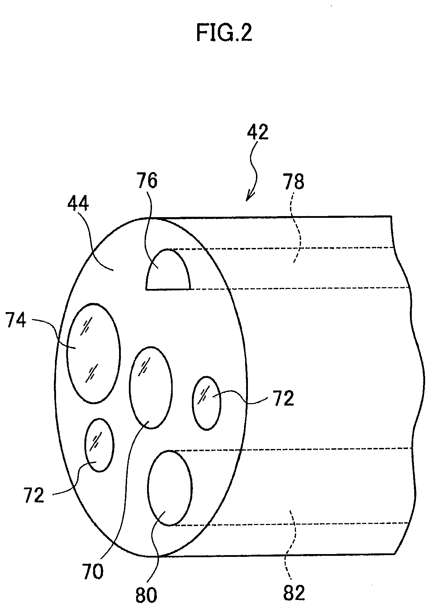

As described later (see Fig. 2), the tube for air supply and water supply 52 communicates with a duct for air supply and water supply provided within the insertion part 14, and sprays air or water from an air-supply and water-supply nozzle provided in a distal end face of a distal end portion 42 to clean an observation window. Further, the tube for constant-pressure air supply 54 communicates with a duct for constant-pressure air supply provided within the insertion part 14, and supplies the carbon dioxide gas into a stomach 16 (lumen) through an opening for constant-pressure air supply provided at the distal end face.

At the time of observation or the like, the carbon dioxide gas is always supplied as gas for constant-pressure air supply. At this time, the air-supply device 56 is provided with a pressure gauge 62 and a flowmeter 64, and the pressure and flow rate of the gas for constant-pressure air supply are measured at predetermined time intervals.

The pressure gauge 62 is originally intended to measure the pressure of the carbon dioxide gas in the stomach 16 (lumen), but a universal pressure gauge is too large to be installed in the distal end portion 42 of the endoscope or the duct of the insertion part 14, and accordingly the pressure gauge 62 is installed in the air-supply device 56 so as to measure the gas pressure through the duct for constant-pressure air supply and the tube for constant-pressure air supply 54 communicating with the stomach 16. Therefore, as compared with a pressure P1 measured by the pressure gauge 62 within the air-supply device 56, an actual pressure P2 in the stomach 16 is low due to pressure loss in the duct on the way, so that, considering the pressure loss (P1 - P2), it is preferred that the air-supply device 56 stores the data of pressure loss (PI - P2) according to the type of an endoscope or the like.

It should be noted that though the pressure gauge 62 is now installed in the air-supply device 56, the pressure gauge 62 may be installed anywhere outside the body of a subject. Further, though the pressure gauge 62 measures the intraluminal pressure through the duct for constant-pressure air supply communicating with the lumen, this measurement may be performed not through the duct for constant-pressure air supply but through, for example, a duct of pressure measurement dedicated to pressure measurement.

On the other hand, the flowmeter 64 measures the flow rate of the gas supplied from the air-supply device 56 to the stomach 16 through the tube for constant-pressure air supply 54 and the duct for constant-pressure air supply of the insertion part 14.

Further, the insertion part 14 is composed of a flexible part 38, a bending part, 40, and a distal end part 42 in this order from the hand operation part 12 on the proximal end side of the endoscope 10 to the distal end side thereof.

The bending part 40 is bent remotely by revolving the pair of angle knobs 28 provided in the hand operation part 12. This allows the operator to direct the distal end part 42 in a desired direction so that the operator can observe different sites in the lumen through an observation window formed in a distal end face of the distal end part 42.

The flexible part 38 connects the hand operation part 12 and the bending part 40 to each other, and is composed of a flexible member so as to be bendable in any direction along the direction of insertion into a subject.

Fig. 2 shows a perspective view of the distal end portion 42 of the insertion part 14.

As shown in Fig. 2, an observation window 70 provided with an imaging device (such as a CCD camera), illumination windows 72 and 72 illuminating an observational range, and a forceps exit 74 are disposed within a distal end face 44 of the distal end part 42. Further, an air-supply/water-supply nozzle 76 and an opening for constant-pressure air supply 80 are disposed in the distal end face 44.

An optical system (observation optical system) for capturing object light in the subject is disposed behind the observation window 70, and the object light representing an observation image captured is received by the CCD, and transmitted to the endoscope processor 200 through a signal cable. Then, the endoscope processor 200 converts the object image into a video signal, thereby displaying the observation image on the monitor device 300 connected with the endoscope processor 200.

The illumination windows 72 and 72 are symmetrically disposed on both sides of the observation window 70, as shown in Fig. 2, and emit illumination light from the light source device 100 to an observation site in the subject. Light from the light source device 100 is introduced into the illumination window 72 through an optical fiber (light guide) disposed in the insertion part 14. Then, the illumination light is emitted through an illumination lens distally disposed and a cover glass fitted in the illumination window 72.

The forceps exit 74 is connected with a forceps channel (not shown) disposed in the insertion part 14, and communicates with the forceps insertion part 30 of the hand operation part 12. Forceps or other various treatment instruments inserted into the forceps entrance 30 expose distal ends of the forceps or other various treatment instruments from the forceps exit 74 through the forceps channel.

The air-supply/water-supply nozzle 76 communicates with a duct for air supply and water supply 78 formed within the insertion part 14. When the observation window 70 becomes dirty, the observation window 70 is cleaned by spraying cleaning liquid and compressed air from the air-supply/water-supply nozzle 76 to the observation window 70. The air-supply/water-supply nozzle 76 sprays a fluid, such as compressed air or cleaning liquid, to the observation window 70 according to air-supply operation and water-supply operation of the air-supply/water-supply button 20 provided in the hand operation part 12. This removes body fluid or dirt attached to the observation window 70 and secures a clear field of view of the observation window 70.

Further, the opening for constant-pressure air supply 80 communicates with the duct for constant-pressure air supply 82. The duct for constant-pressure air supply 82 communicates with the tube for constant-pressure air supply 54, and the carbon dioxide gas adjusted to a predetermined pressure by the air-supply device 56 is supplied from the duct for constant-pressure air supply 82 to a lumen, such as the stomach 16, through the opening for constant-pressure air supply 80. It should be noted that in this text such air supply is referred to as constant-pressure air supply.

During observation of the lumen, while the gas pressure is being measured by the pressure gauge 62 within the air-supply device 56, constant-pressure air supply to the lumen is always performed so as to keep the lumen at a predetermined pressure.

As described above, in the first embodiment, pressure measurement performed by the pressure gauge 62 is performed by measuring gas pressure in the tube for constant-pressure air supply 54 communicating with the stomach 16 through the duct for constant-pressure air supply, but, as described later, another duct, for example, a duct for pressure measurement dedicated to pressure measurement may be used for the pressure measurement performed by the pressure gauge 62.

Thus, since the pressure in the lumen is not directly measured, but measured through the duct communicating with the lumen, precise measurement of the pressure P2 in the lumen (in the stomach 16) requires the stomach 16 and the tube for constant-pressure air supply 54 to communicate with each other with the duct for constant-pressure air supply 82 unobstructed and with the opening for constant-pressure air supply 80 opened in the same state.

However, high-viscosity body fluids (digestive juice, blood, and the like) are present in the human lumens, and these body fluids attach to the distal end face 44 to obstruct an opening portion of the duct for constant-pressure air supply 82 (the opening for constant-pressure air supply 80) completely or partially, which results in the possibility that an opening state of the opening for constant-pressure air supply 80 may change. Even if the pressure is measured with the opening state changed, precise pressure measurement cannot be performed. Therefore, in the present invention, constant-pressure air-supply control as described later is performed so that precise pressure measurement is achieved.

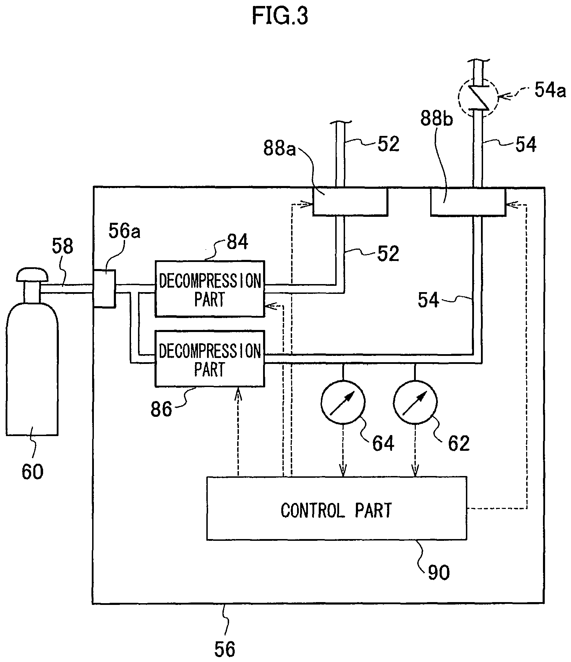

Fig. 3 shows the configuration of the air-supply device 56 that performs the constant-pressure air-supply control.

As shown in Fig. 3, the air-supply device 56 includes decompression parts 84 and 86, an on-off valve 88a provided in the tube for air supply and water supply 52 connected with the decompression part 84, an on-off valve 88b provided in the tube for constant pressure air supply 54 connected with the decompression part 86, and a control part 90 which controls the decompression parts 84 and 86, the on-off valves 88a and 88b, and the like.

The decompression parts 84 and 86 are supplied with the high-pressure carbon dioxide gas from the tube for high-pressure gas 58 connected with the carbon dioxide gas cylinder 60 through a high-pressure connector 56a. Further, the tube for constant-pressure air supply 54 is provided with the pressure gauge 62 and the flowmeter 64, which measure the pressure of the gas vaporized from the carbon dioxide gas cylinder 60, decompressed to a predetermined pressure by the decompression part 86, and supplied to the tube for constant-pressure air supply 54. The respective measurement results of the pressure gauge 62 and the flowmeter 64 are sent to the control part 90.

Each of the decompression parts 84 and 86 decompress the carbon dioxide gas supplied through the high-pressure connector 56a to a predetermined pressure based on a control signal from the control part 90. Further, each of the on-off valves 88a and 88b opens or closes according to a control signal from the control part 90. The control part 90 controls the decompression part 86 and the on-off valve 88b based on the measurement results of the pressure gauge 62 and the flowmeter 64, thereby performing constant-pressure air-supply control. A pressure gauge and a flowmeter not shown in the figures may be connected with the tube for air supply and water supply 52, thereby detecting an air-supply pressure and an air-supply amount.

The constant-pressure air-supply control according to the present invention forces the gas to be supplied to the duct for constant-pressure air supply 82 before pressure measurement separately from the constant-pressure air supply described above, thereby removing attached matter, such as liquid attached to the inside of the duct for constant-pressure air supply 82. Such air supply is referred to as flushing or duct flushing air supply. Such flushing is intended to make the pressure in a lumen, such as the stomach 16, and the pressure in the tube for constant-pressure air supply 54 which is to be measured substantially equal to each other, thereby measuring precise intraluminal pressure.

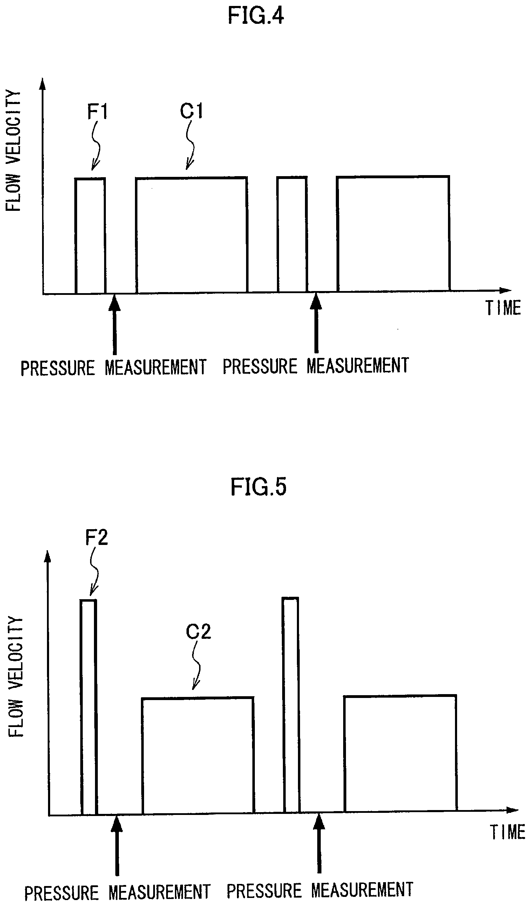

Fig. 4 shows a first constant-pressure air-supply control method.

The diagram (graph) shown in Fig. 4 is a timing chart showing the duct flushing air supply and the constant-pressure air supply, showing time on the horizontal axis and the flow rate of constant-pressure air supply gas on the vertical axis.

Here, the flow rate is a flow rate of gas for constant-pressure air supply (carbon dioxide gas) vaporized from the carbon dioxide gas cylinder 60, decompressed to a predetermined pressure at the decompression part 86, and supplied to the tube for constant-pressure air supply 54.

As shown in Fig. 4, constant-pressure air supply C1 is performed at certain time intervals, and pressure measurement is performed before the constant-pressure air supply C1. Further, duct flushing air supply F1 is performed necessarily before pressure measurement. In particular, in order to perform precise pressure measurement, it is preferred that duct flushing air supply is performed immediately before pressure measurement. Therefore, the control part 90 not only issues an instruction to perform pressure measurement, but also instructs a flushing device composed of the decompression part 86 and the on-off valve 88b to perform flushing in synchronization with the pressure measurement, as shown in Fig. 4. At this time, the control part 90 issues an instruction to perform pressure measurement during a period for which the constant-pressure air supply C1 is not performed and the gas supply is stopped, and issues an instruction to perform supply of gas for flushing, thereby performing flushing before the pressure measurement, as shown in Fig. 4.

This duct flushing air supply F1 in the first constant-pressure air-supply control supplies a flow rate of at least equal to the volume of the duct for constant-pressure air supply 82. At this time, if mucus or the like is attached to the opening portion of the duct for constant-pressure air supply 82 (the inside of the opening for constant-pressure air supply 80), the duct flushing air supply F1 may be performed at a higher pressure than the constant-pressure air supply to increase the flow velocity of air supply, thereby blowing off the mucus or the like.

Further, even if the duct flushing air supply F1 is performed at a flow rate of equal to or more than the volume of the duct for constant-pressure air supply 82 in this manner, the volume of the duct for constant-pressure air supply 82 is much smaller than that of the lumen, for example, the volume of the duct is 15cc when the volume of the stomach is 1500cc (in this example, the volume of the duct is 1/100 of the volume of the lumen). Therefore, there is not the possibility that the duct flushing air supply causes a sharp increase in the pressure of the lumen, resulting in a strain on the subject.

Here, it is preferred that the flow rate supplied by the duct flushing air supply F1 is at least equal to the volume of the duct for constant-pressure air supply 82, and it is only necessary to supply a flow rate approximately corresponding to the volume of a duct which is composed of the tube for constant-pressure air supply 54 and the duct for constant-pressure air supply 82 and through which constant-pressure air-supply gas is supplied from the air-supply device 56 to the opening for constant-pressure air supply 80.

It should be noted that, as shown by broken line in Fig. 3, a check valve 54a may be provided in the tube for constant-pressure air supply 54 communicating with the duct for constant-pressure air supply 82 so that duct flushing air supply is performed with a flow rate of constant-pressure air-supply gas equal to or more than the volume of a duct from the check valve 54a to the opening for constant-pressure air supply 80.

Thus, since the duct flushing air supply F1 removes liquid or the like attached to the inside of the duct for constant-pressure air supply 82 before (in particular, immediately before) pressure measurement, thereby making the lumen and the tube for constant-pressure air supply 54 whose pressure is intended to be measured to communicate completely with each other so that the pressure of the tube for constant-pressure air supply 54 becomes substantially equal to the pressure of the lumen, and then the pressure measurement is performed by the pressure gauge 62, the pressure of the lumen can be precisely measured.

Fig. 5 shows a second constant-pressure air-supply control method.

Fig. 5 is a timing chart showing the duct flushing air supply and the constant-pressure air supply in the second constant-pressure air-supply control.

Also in the second constant-pressure air-supply control, like the first constant-pressure air supply described above, constant-pressure air supply C2 is performed at certain time intervals, and pressure measurement is performed before each constant-pressure air supply C2. Further, duct flushing air supply F2 is performed necessarily before every pressure measurement. Also in this case, it is preferred that the duct flushing air supply F2 is performed immediately before pressure measurement.

The duct flushing air supply F2 in the second constant-pressure air supply control is performed for a shorter period of time than the duct flushing air supply F1 in the first constant-pressure air supply, as shown in Fig. 5, but has an increased flow velocity, thereby securing the same flow rate as the duct flushing air supply F1 of the first constant-pressure air supply.

Thus, in the second constant-pressure air-supply control, brief flushing air supply with a higher flow velocity removes liquid or the like attached to the inside of the duct for constant-pressure air supply 82.

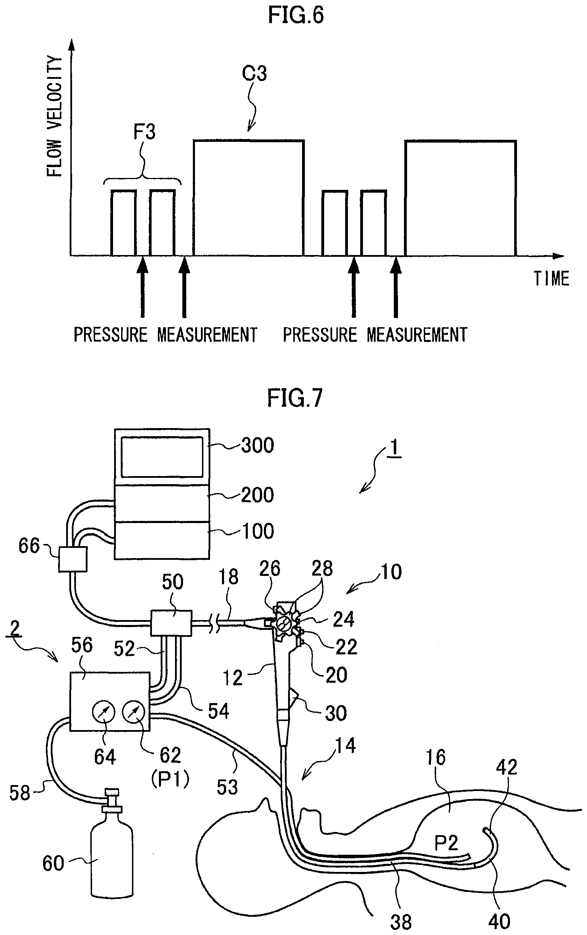

Fig. 6 shows a third constant-pressure air-supply control method.

Fig. 6 is a timing chart showing the duct flushing air supply and the constant-pressure air supply in the third constant-pressure air-supply control.

Also in the third constant-pressure air-supply control, constant-pressure air supply C3 is performed at certain time intervals, duct flushing air supply F3 is performed before each constant-pressure air supply C3, and then pressure measurement is performed, but, in the example shown in Fig. 6, the duct flushing air supply F3 and pressure measurement are performed in twice.

The duct flushing air supply F3 in this case is lower in flow velocity than the constant-pressure air supply C3 in each time, and also lower in flow rate, but even with a flow rate equal to or less than the volume of the duct for constant-pressure air supply 82, pressure measurement is performed in plural times, and the measurement results are compared with each other to detect the presence or absence of liquid or the like in the duct for constant-pressure air supply 82, so that precise pressure measurement can be performed avoiding a pressure measurement error.

For example, in Fig. 6, the measurement result of the first pressure measurement of the two pressure measurements is represented by S1, the measurement result of the second pressure measurement is represented by S2, and when a difference between these measurement results (S1-S2) is equal to or less than a predetermined value, it is determined that the pressure measurement has been performed precisely, and when the difference (S1-S2) is higher than the predetermined value, it is determined that a pressure measurement error has occurred, on the assumption that the state of the duct has changed due to the presence of liquid in the duct for constant-pressure air supply 82.

In the example shown in Fig. 6, pressure measurement is performed twice, but the number of times of pressure measurement is not limited to twice, and pressure measurement may be performed any number of times except once. Actually, it is preferred that duct flushing air supply and pressure measurement are repeated until the difference between the measurement results becomes less than the predetermined value.

For example, during a period of stopping gas supply from the air-supply device (gas supply device) 56, the control part 90 determines whether or not a difference between the result of Nth (here, N is a natural number equal to or more than 1) pressure measurement and the result of (N + 1)th pressure measurement is less than a predetermined threshold, and the control part 90 issues an instruction to repeat the flushing and pressure measurement described above until it is determined that the difference is less than the predetermined threshold.

In the first embodiment described above, pressure measurement is performed through the tube for constant-pressure air supply 54 communicating with the duct for constant-pressure air supply 82, but, as described later, a duct for pressure measurement may be provided separately from the duct for constant-pressure air supply 82 so that pressure measurement is performed through the duct for pressure measurement.

Fig. 7 is a configuration diagram showing the outline of an endoscopic system provided with an endoscope air-supply system according to a second embodiment of the present invention.

In Fig. 7, similar components to those in the first embodiment shown in Fig. 1 are denoted by identical reference signs. As shown in Fig. 7, in the second embodiment, separately from the tube for constant-pressure air supply 54, a duct dedicated to pressure measurement (duct for pressure measurement) 53 is drawn from the air-supply device 56, and inserted into the lumen along the endoscope insertion part 14 so that the pressure in the lumen is measured through the duct for pressure measurement 53.

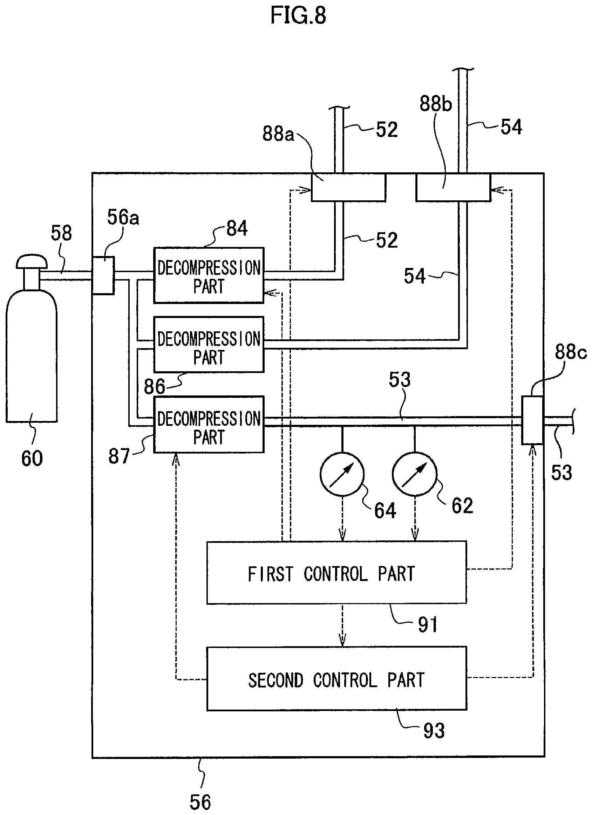

Fig. 8 is a configuration diagram showing the outline of an air-supply device 56 of the second embodiment.

As shown in Fig. 8, the duct for pressure measurement 53 is connected to a decompression part 87, and the decompression part 87 is connected to a carbon dioxide gas cylinder 60 through a high-pressure connector 56a. Further, an on-off valve 88c is provided at an outlet of the air-supply device 56 of the duct for pressure measurement 53. Further, the pressure gauge 62 and the flowmeter 64 are disposed between the on-off valve 88c and the decompression part 87 in the duct for pressure measurement 53.

The air-supply device 56 is provided with a first control part 91 which controls supply of the gas for constant-pressure air supply based on the result of pressure measurement, and a second control part 93 which controls the flow velocity or flow rate of the gas for flushing based on a condition of supply of the gas for constant-pressure air supply. Though controlled by the control part 90 obtained by unifying these two control parts 91 and 93 in the first embodiment, the supply of the gas for constant-pressure air supply and the supply of the gas for flushing may be controlled by different control parts in this manner.

In the second embodiment, the first control part 91 issues a pressure-measurement instruction, and issues a flushing instruction through the second control part 93. The second control part 93 controls the decompression part 87 and the on-off valve 88c so as to perform flushing. After flushing, the result of measurement obtained by the pressure gauge 62 is inputted into the first control part 91, and the first control part 91 controls supply of the gas for constant-pressure air supply based on the measurement result. On the other hand, the second control part 93 receives the condition of supply of the gas for constant-pressure air supply from the first control part, and controls the flow velocity and flow rate of the gas for flushing based on the condition.

It should be noted that, in a case where the present invention is used in a therapy with excision of a lumen wall through a lumen or an intra-abdominal therapy with penetration of a lumen wall, the duct for pressure treatment 53 may be inserted into an abdominal cavity through a trocar to measure the pressure.

Further, as described later, a supply line of the gas for constant-pressure air supply and a supply line of the gas for flushing may be completely separated from each other so as to be supplied with gas from different carbon dioxide gas cylinders.

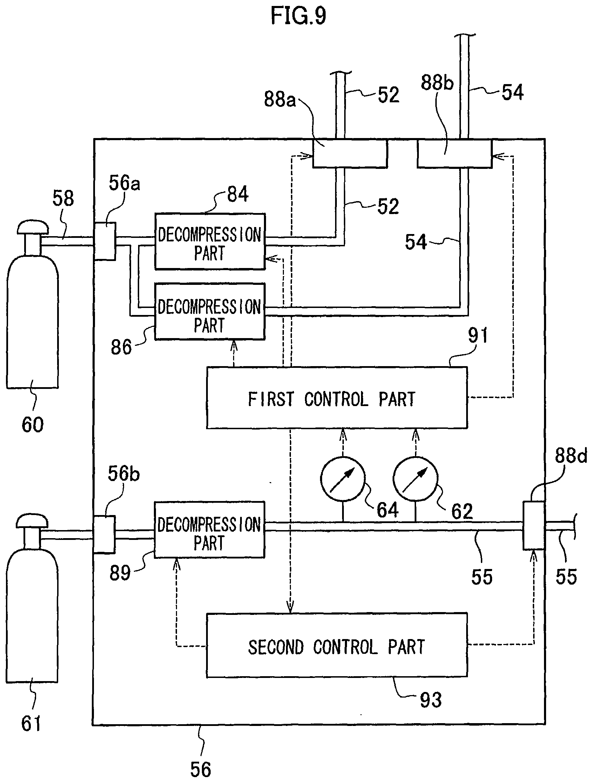

Fig. 9 shows the air-supply device 56 of a modification of the second embodiment.

In the modification shown in Fig. 9, a duct for pressure measurement 55 is provided completely separately from the tube for constant-pressure air supply 54 communicating with the duct for constant-pressure air supply 82, and connected with another carbon dioxide gas cylinder 61 through a decompression part 89. Then, this duct for pressure measurement 55 is provided with the pressure gauge 62 and the flowmeter 64 between the decompression part 89 and the on-off valve 88d.

The decompression part 89 and the on-off valve 88d, which constitute a flushing device, are controlled by the second control part 93 so as to perform flushing before pressure measurement. Further, the result of pressure measurement obtained by the pressure gauge 62 is sent to the first control part 91, and the first control part 91 controls supply of the gas for constant-pressure air supply based on the result of pressure measurement.

Hereinabove, some examples of the constant-pressure air-supply control has been described, and each of these examples can measure pressure in a lumen precisely, since duct flushing air supply is used to remove liquid or the like attached to the inside of a duct for pressure measurement, thereby causing the duct for pressure measurement to communicate completely with the lumen so that the pressure gauge 62 measures the pressure of the duct made substantially equal to the pressure of the lumen.

Further, since the gas pressure in the lumen is always measured precisely in this manner, automatic constant-pressure air supply can be made so as to keep the pressure in the lumen appropriate, and therefore the operator does not need to operate the air-supply button frequently manually.

It should be noted that if the monitor device (display device) 300 is configured to display whether the air-supply device 56 is currently in constant-pressure air supply or in duct flushing air supply, the operator can always know an air-supply state of the air-supply device 56, which results in improvement of operation efficiency.

Further, in the embodiments described above, an opening for the duct for constant-pressure air supply, like the opening 80 for constant-pressure air supply 80 (see Fig. 2), is provided in the distal end face 44 of the endoscope, but the position of installment of the opening for the duct for constant-pressure air supply is not necessarily limited to the distal end face 44.

Further, the duct for constant-pressure air supply 82 is provided within the endoscope (within the insertion part 14) in the embodiments, but in a case where an endoscope and an overtube are combined, a duct for constant-pressure air supply (a lumen for constant-pressure air supply) may be a space between the endoscope and the overtube created when the endoscope is inserted into the overtube, or a lumen in a multi-lumen overtube or the like, which the endoscope cannot be passed through, may be used.

Further, in a case where different ducts for constant-pressure air supply are used in this manner, for example, a memory part may be provided in the air-supply device 56 so as to preliminarily register (store) the respect volumes (or luminal diameters) of ducts for constant-pressure air supply so that the flow rate of duct flushing air supply can be selected according to the volume or diameter of a duct for constant-pressure air supply to be used.

Alternatively, the flow rate of duct flushing may be automatically switched by inputting endoscope information.

The present invention is described as being used for observation or treatment in a lumen, but the present invention may be used for a therapy to excise a wall of lumen through a lumen or an intra-abdominal therapy with penetration of a wall of lumen.

Though the endoscope air-supply systems according to the present invention have been described above in detail, the present invention is not limited to the above embodiments, and obviously can be modified or varied variously without departing from the scope of the invention, which is defined in the claims.

## Claims

1. An endoscope air-supply system (2) comprising:
a gas supply device (56) and an air-supply duct adapted to supply predetermined gas to a lumen of a subject through the air-supply duct;
a duct for pressure measurement (53,55) communicating with the lumen;
a pressure measurement device (62) adapted to measure pressure in the lumen and connected through the duct for pressure measurement (53,55) communicating with the lumen;
a flushing device (86, 88b) adapted to supply gas for flushing to the duct for pressure measurement (53,55); and
an instruction device (90) adapted to instruct the pressure measurement device to perform pressure measurement and to instruct the flushing device to supply the gas for flushing in synchronism with the pressure measurement by the pressure measurement device (62),
**characterized by**
the instruction device is adapted to instruct the pressure measurement device to perform pressure measurement during stoppage of gas supply by the gas supply device, and to instruct the flushing device to supply the gas for flushing before the pressure measurement device performs pressure measurement.

2. The endoscope air-supply system (2) according to claim 1, wherein the flushing device is adapted to supply an amount of the gas for flushing equivalent to or more than the volume of the duct for pressure measurement (53,55).

3. The endoscope air-supply system (2) according to claim 2, wherein
the duct for pressure measurement (53,55) is provided with a check valve (54a) which prevents backflow of fluid from the lumen, and
the flushing device is adapted to supply the gas for flushing which is at least equal to a volume from the check valve (54a) to an opening portion on the side of the lumen of the duct for pressure measurement.

4. The endoscope air-supply system (2) according to any one of claims 1 to 3, wherein the duct for pressure measurement (53,55) is composed of at least a portion of the air-supply duct.

5. The endoscope air-supply system (2) according to any one of claims 1 to 4, wherein the gas for flushing supplied by the flushing device is gas supplied from the same gas supply source as the gas supplied by the gas supply device.

6. The endoscope air-supply system (2) according to any one of claims 1 to 5, further comprising a first control device (91) adapted to control gas supply performed by the gas supply device based on the result of the measurement by the pressure measurement device.

7. The endoscope air-supply system (2) according to any one of claims 1 to 6, further comprising a second control device (93) adapted to control the flow velocity or the flow rate of the gas for flushing supplied by the flushing device based on a condition of supply of the gas supplied by the gas supply device.

8. The endoscope air-supply system (2) according to claim 7, wherein the second control device (93) is adapted to control the flow velocity of the gas for flushing supplied by the flushing device so as to be equal to the flow velocity of the gas supplied by the gas supply device.

9. The endoscope air-supply system (2) according to claim 7, wherein the second control device (93) is adapted to control the flow velocity of the gas for flushing supplied by the flushing device so as to be faster than the flow velocity of the gas supplied by the gas supply device.

10. The endoscope air-supply system (2) according to any one of claims 1 to 9, wherein the instruction device (90) is adapted to issue an instruction to repeat supply of the gas for flushing by the flushing device and pressure measurement by the pressure measurement device in plural times during stoppage of gas supply by the gas supply device.

11. The endoscope air-supply system (2) according to claim 10, further comprising a determination device adapted to determinate whether or not a difference between the result of Nth pressure measurement and the result of (N + 1)th pressure measurement is less than a predetermined threshold (where N is a natural number equal to or more than 1) during stoppage of gas supply by the gas supply device, wherein
the instruction device (90) is adapted to issue an instruction to repeat supply of the gas for flushing by the flushing device and pressure measurement by the pressure measurement device until the determination device determines that the difference is less than the predetermine threshold.

12. The endoscope air-supply system (2) according to any oe of claims 1 to 11, further comprising a display device (300) adapted to confirm whether or not supply of the gas for flushing by the flushing device or gas supply by the gas supply device is being performed.

## Patentansprüche

1. Luftzuführsystem (2) für ein Endoskop, umfassend:
eine Gaszuführvorrichtung (56) und eine Luftzuführleitung, ausgebildet zum Zuführen vorbestimmten Gases zu einem Lumen eines Subjekts durch die Luftzuführleitung;
eine mit dem Lumen kommunizierende Leitung für Druckmessung (53, 55);
eine Druckmessvorrichtung (62), ausgebildet zum Messen von Druck in dem Lumen und über die mit dem Lumen kommunizierende Leitung für Druckmessung (53, 55) angeschlossen;
eine Spülvorrichtung (86, 88b), ausgebildet zum Zuführen von Gas zum Spülen der Leitung für Druckmessung (53, 55); und
eine Befehlsvorrichtung (90), ausgebildet zum Anweisen der Druckmessvorrichtung, eine Druckmessung auszuführen, und zum Anweisen der Spülvorrichtung, das Gas zum Spülen zuzuführen, synchron mit der Druckmessung durch die Druckmessvorrichtung (62),
**dadurch gekennzeichnet,**
**dass** die Befehlsvorrichtung ausgebildet ist zum Anweisen der Druckmessvorrichtung, eine Druckmessung während des Anhaltens der Gaszufuhr durch die Gaszuführvorrichtung auszuführen, und um die Spülvorrichtung anzuweisen, das Gas zum Spülen zuzuführen, bevor die Druckmessvorrichtung eine Druckmessung vornimmt.

2. Luftzuführsystem (2) für ein Endoskop nach Anspruch 1,
bei dem die Spülvorrichtung ausgebildet ist zum Zuführen einer Gasmenge zum Spülen äquivalent zu oder mehr als das Volumen der Leitung für Druckmessung (53, 55).

3. Luftzuführsystem (2) für ein Endoskop nach Anspruch 2,
bei dem die Leitung für Druckmessung (53, 55) mit einem Rückschlagventil (54a) ausgestattet ist, das einen Rückstrom von Fluid aus dem Lumen unterbindet, und
die Spülvorrichtung ausgebildet ist zum Zuführen des Gases zum Spülen mindestens gleich einem Volumen von dem Rückschlagventil (54a) zu einem Öffnungsbereich auf der Seite des Lumens der Leitung für Druckmessung.

4. Luftzuführsystem (2) für ein Endoskop nach einem der Ansprüche 1 bis 3,
bei dem die Leitung für Druckmessung (53, 55) sich zumindest aus einem Abschnitt der Luftzuführleitung zusammensetzt.

5. Luftzuführsystem (2) für ein Endoskop nach einem der Ansprüche 1 bis 4,
bei dem das Gas zum Spülen, das von der Spülvorrichtung geliefert wird, Gas ist, welches von derselben Gaszuführquelle geliefert wird wie das von der Gaszuführvorrichtung zugeführte Gas.

6. Luftzuführsystem (2) für ein Endoskop nach einem der Ansprüche 1 bis 5,
weiterhin umfassend eine erste Steuereinrichtung (91), ausgebildet zum Steuern der Gaszufuhr, die von der Gaszuführvorrichtung vorgenommen wird, basierend auf dem Ergebnis der Druckmessung durch die Druckmessvorrichtung.

7. Luftzuführsystem (2) für ein Endoskop nach einem der Ansprüche 1 bis 6,
weiterhin umfassend eine zweite Steuereinrichtung (93), ausgebildet zum Steuern der Strömungsgeschwindigkeit oder des Strömungsdurchsatzes des Gases zum Spülen, das von der Spülvorrichtung geliefert wird, basierend auf einem Zustand der Zufuhr von Gas, das von der Gaszuführvorrichtung zugeführt wird.

8. Luftzuführsystem (2) für ein Endoskop nach Anspruch 7,
bei dem die zweite Steuereinrichtung (93) ausgebildet ist zum Steuern der Strömungsgeschwindigkeit des Gases zum Spülen, das von der Spülvorrichtung geliefert wird, so dass sie gleich der Strömungsgeschwindigkeit des von der Gaszuführvorrichtung gelieferten Gases ist.

9. Luftzuführsystem (2) für ein Endoskop nach Anspruch 7,
bei dem die zweite Steuereinrichtung (93) ausgebildet ist zum Steuern der Strömungsgeschwindigkeit des Gases zum Spülen, das von der Spülvorrichtung zugeführt wird, derart, dass sie höher ist als die Strömungsgeschwindigkeit des von der Gaszuführvorrichtung zugeführten Gases.

10. Luftzuführsystem (2) für ein Endoskop nach einem der Ansprüche 1 bis 9,
bei dem die Befehlsvorrichtung (90) ausgebildet ist zum Abgeben eines Befehls zum Wiederholen der Zufuhr des Gases zum Spülen durch die Spülvorrichtung und einer Druckmessung durch die Druckmessvorrichtung in wiederholter Weise während des Anhaltens der Gaszufuhr durch die Gaszuführvorrichtung.

11. Luftzuführsystem (2) für ein Endoskop nach Anspruch 10,
weiterhin umfassend eine Bestimmungseinrichtung, ausgebildet zum Feststellen, ob eine Differenz zwischen dem Ergebnis einer N-ten Druckmessung und dem Ergebnis einer (N +1)-ten Druckmessung kleiner ist als ein vorbestimmter Schwellenwert (wobei N eine natürliche Zahl gleich oder größer 1 ist), während die Gaszufuhr durch die Gaszuführvorrichtung anhält, wobei
die Befehlsvorrichtung (90) ausgebildet ist zum Ausgeben eines Befehls zum Wiederholen der Zufuhr von Gas zum Spülen durch die Spülvorrichtung und der Druckmessung durch die Druckmessvorrichtung, bis die Bestimmungseinrichtung feststellt, dass die Differenz kleiner ist als der vorbestimmte Schwellenwert.

12. Luftzuführsystem (2) für ein Endoskop nach einem der Ansprüche 1 bis 11,
weiterhin umfassend eine Anzeigevorrichtung (300), ausgebildet zum Bestätigen, ob die Zufuhr des Gases zum Spülen durch die Spülvorrichtung oder die Gaszufuhr durch die Gaszuführvorrichtung ausgeführt wird oder nicht.

## Revendications

1. Système d'alimentation en air d'endoscope (2), comprenant :
un dispositif d'alimentation en gaz (56) et un conduit d'alimentation en air apte à alimenter un gaz prédéterminé vers une lumière d'un sujet par le biais du conduit d'alimentation en air ;
un conduit pour mesure de pression (53, 55) communiquant avec la lumière ;
un dispositif de mesure de pression (62) apte à mesurer la pression dans la lumière et connecté par le biais du conduit pour mesure de pression (53, 55) communiquant avec la lumière ;
un dispositif de chasse (86, 88b) apte à alimenter un gaz de chasse vers le conduit pour mesure de pression (53, 55), et
un dispositif d'instruction (90) apte à ordonner au conduit de mesure de pression de réaliser la mesure de pression et ordonner au dispositif de chasse d'alimenter le gaz de chasse de manière synchrone avec la mesure de chasse par le dispositif de mesure de pression (62),
**caractérisé par le fait que**
le dispositif d'instruction est apte à ordonner au dispositif de mesure de pression pour de réaliser la mesure de pression durant l'arrêt de l'alimentation en gaz par le dispositif d'alimentation en gaz, et à ordonner au dispositif de chasse d'alimenter le gaz de chasse avant que le dispositif de mesure de pression ne réalise la mesure de pression.

2. Système d'alimentation en air d'endoscope (2) selon la revendication 1, dans lequel le dispositif de chasse est apte à fournir une quantité du gaz de chasse équivalente ou supérieure au volume du conduit pour mesure de pression (53, 55).

3. Système d'alimentation en air d'endoscope (2) selon la revendication 2, dans lequel le conduit pour mesure de pression (53, 55) est doté d'un clapet antiretour (54a), lequel empêche le retour du fluide à partir de la lumière, et
le dispositif de chasse est apte à alimenter le gaz de chasse, lequel est au moins égal à un volume allant du clapet antiretour (54a) à une portion d'ouverture sur le côté de la lumière du conduit pour mesure de pression.

4. Système d'alimentation en air d'endoscope (2) selon l'une quelconque des revendications 1 à 3, dans lequel le conduit pour mesure de pression (53, 55) est composé d'au moins une portion du conduit d'alimentation en air.

5. Système d'alimentation en air d'endoscope (2) selon l'une quelconque des revendications 1 à 4, dans lequel le gaz de chasse alimenté par le dispositif de chasse est un gaz alimenté à partir de la même source d'alimentation en gaz que pour le gaz alimenté par le dispositif d'alimentation en gaz.

6. Système d'alimentation en air d'endoscope (2) selon l'une quelconque des revendications 1 à 5, comprenant en outre un premier dispositif de commande (91) apte à commander l'alimentation en gaz réalisée par le dispositif d'alimentation en gaz sur la base du résultat de la mesure par le dispositif de mesure de pression.

7. Système d'alimentation en air d'endoscope (2) selon l'une quelconque des revendications 1 à 6, comprenant en outre un second dispositif de commande (93) apte à commander la vitesse d'écoulement ou le débit du gaz de chasse alimenté par le dispositif de chasse sur la base d'une condition d'alimentation du gaz alimenté par le dispositif d'alimentation en gaz.

8. Système d'alimentation en air d'endoscope (2) selon la revendication 7, dans lequel le second dispositif de commande (93) est apte à commander la vitesse d'écoulement du gaz de chasse alimenté par le dispositif de chasse de manière à ce qu'elle soit égale à la vitesse d'écoulement du gaz alimenté par le dispositif d'alimentation en gaz.

9. Système d'alimentation en air d'endoscope (2) selon la revendication 7, dans lequel le second dispositif de commande (93) est apte à commander la vitesse d'écoulement du gaz de chasse alimenté par le dispositif de chasse de manière à ce qu'elle soit plus rapide que la vitesse d'écoulement du gaz alimenté par le dispositif d'alimentation en gaz.

10. Système d'alimentation en air d'endoscope (2) selon l'une quelconque des revendications 1 à 9, dans lequel le dispositif d'instruction (90) est apte à émettre une instruction afin de répéter l'alimentation du gaz de chasse par le dispositif de chasse et la mesure de pression par le dispositif de mesure de pression plusieurs fois durant l'arrêt de l'alimentation en gaz par le dispositif d'alimentation en gaz.

11. Système d'alimentation en air d'endoscope (2) selon la revendication 10, comprenant en outre un dispositif de détermination apte à déterminer si oui ou non une différence entre le résultat de la nième mesure de pression et le résultat de la (N+1)ième mesure de pression est inférieure à un seuil prédéterminé (où N est un nombre naturel supérieur ou égal à 1), durant l'arrêt de l'alimentation en gaz par le dispositif d'alimentation en gaz, dans lequel
le dispositif d'instruction (90) est apte à émettre une instruction afin de répéter l'alimentation du gaz de chasse par le dispositif de chasse et la mesure de pression par le dispositif de mesure de pression jusqu'à ce que le dispositif de détermination détermine que la différence est inférieure au seuil prédéterminé.

12. Système d'alimentation en air d'endoscope (2) selon l'une quelconque des revendications 1 à 11, comprenant en outre un dispositif d'affichage (300) apte à confirmer si oui ou non l'alimentation en gaz de chasse par le dispositif de chasse ou l'alimentation en gaz par le dispositif d'alimentation en gaz est en cours de réalisation.
